# FASCICULE DE BREVET EUROPEEN

(11) **EP 3 145 506 B1**
(45) Date de publication et mention de la délivrance du brevet: **13.03.2019**
(21) Numéro de dépôt: 15732301.5
(22) Date de dépôt: 12.05.2015
(51) Int. Cl.: A61K 31/355, A61K 9/12, A61K 33/00, A61K 9/72, A61P 25/16, A61P 25/28

(54) **ASSOCIATION DE XÉNON ET D'UN ANTIOXYDANT POUR LUTTER CONTRE UNE MALADIE NEURODÉGÉNÉRATIVE DE TYPE MALADIE DE PARKINSON**
KOMBINATION AUS XENON UND EINEM ANTIOXIDANS ZUR STEUERUNG VON NEURODEGENERATIVEN ERKRANKUNGEN VOM PARKINSON-TYP
COMBINATION OF XENON AND AN ANTIOXIDANT TO CONTROL A PARKINSON'S DISEASE-TYPE NEURODEGENERATIVE DISEASE

(30) Priorité: 21.05.2014 FR 1454586
(43) Date de publication de la demande: 29.03.2017
(73) Titulaire: L'Air Liquide Société Anonyme pour l'Etude et l'Exploitation des Procédés Georges Claude, 75007 Paris (FR); L'Institut du Cerveau et de la Moelle Epiniere, 75013 Paris (FR)
(72) Inventeur: LAVAUR, Jérémie, 75007 Paris (FR); HIRSCH, Etienne, 78000 Versailles (FR); MICHEL, Patrick, 75004 Paris (FR); LEMAIRE, Marc, 75014 Paris (FR)
(74) Mandataire: Pittis, Olivier
(86) Numéro de dépôt international: PCT/FR2015/051241
(87) Numéro de publication internationale: WO 2015/177435

(56) Documents cités:
- EP-A1- 1 552 840
- WO-A1-2014/057179
- FR-A1- 2 952 305
- ETMINAN M ET AL: "Intake of vitamin E, vitamin C, and carotenoids and the risk of Parkinson's disease: a meta-analysis", LANCET NEUROLOGY, LANCET PUBLISHING GROUP, LONDON, GB, vol. 4, no. 6, 1 juin 2005 (2005-06-01), pages 362-365, XP027725351, ISSN: 1474-4422 [extrait le 2005-06-01]

## Description

L'invention porte sur une composition médicamenteuse comprenant en combinaison du xénon gazeux et un antioxydant choisi parmi la vitamine E et le TROLOX, utilisée pour traiter, ralentir ou prévenir une dégradation neurologique, en rapport avec, ou consécutive à une maladie neurodégénérative, en particulier la maladie de Parkinson.

Les récepteurs/canaux NMDA (pour N-Méthyl-D-Aspartate) sont des entités moléculaires de la membrane plasmique des cellules neuronales. Ces récepteurs, sont la cible des molécules de glutamate libérées dans l'espace synaptique et extra-synaptique, le glutamate étant un neurotransmetteur excitateur assurant la communication d'une cellule nerveuse à une autre.

Certaines maladies neurodégénératives, en particulier la maladie de Parkinson, se caractérisent par la mort des neurones dopaminergiques du tronc cérébral et se manifestent cliniquement par une perte du contrôle de la motricité volontaire, des tremblements, une bradykinésie et une rigidité.

La dégénérescence neuronale dans la maladie de Parkinson met vraisemblablement en jeu une excitotoxicité, c'est-à-dire un mécanisme lié à une sur-stimulation des récepteurs NMDA provoquée par un excès du neurotransmetteur glutamate dans l'espace extracellulaire (Metha et coll., Eur. J. Pharmacol, 2013).

En effet, il a été montré dans des modèles cellulaires et animaux mimant la maladie de Parkinson qu'un dysfonctionnement des transporteurs des amino-acides excitateurs (EAATs) portés par les cellules gliales et les neurones, conduit à une mort cellulaire dopaminergique (Nafia et al, J Neurochem, 2008; Swanson et al, Ann Neurol, 2011).

De plus, les neurones dopaminergiques en culture sont tout particulièrement vulnérables lorsqu'ils subissent un stress causé par du glutamate exogène (Douhou et al, J Neurochem, 2001).

Par ailleurs, après lésion du système dopaminergique chez le rat et le singe, il y a augmentation de l'activité des neurones sous-thalamiques qui sont à l'origine de l'innervation excitatrice glutamatergique des neurones dopaminergiques (Wallace et al, Brain, 2007).

En outre, il a été établi que la dopamine (DA) protège de la toxicité induite par le glutamate (Vaarmann et al, Cell Death and Disease, 2013). Ainsi, une déplétion en DA causée par une lésion dopaminergique, pourrait favoriser en soi la vulnérabilité des neurones dopaminergiques au stress excitotoxique.

EP1552840 divulgue une combinaison de xénon et de dioxyde de carbone pour protéger les cellules, tissus et organes dans des états consécutifs à une maladie neuro-dégénérative.

Toutefois, il n'existe pas, à ce jour, de médicament réellement efficace et satisfaisant au plan thérapeutique permettant de traiter, ralentir et/ou prévenir une dégradation neurologique consécutive à une maladie neurodégénérative, en particulier la maladie de Parkinson, chez un être humain.

Au vu de cela, le problème qui se pose est de proposer un médicament permettant de traiter, ralentir et/ou prévenir une dégradation neurologique consécutive à une maladie neurodégénérative, en particulier la maladie de Parkinson, chez un être humain.

La solution selon la présente invention est une combinaison médicamenteuse comprenant du xénon gazeux et au moins un antioxydant sous forme liquide ou solide pour une utilisation pour traiter, ralentir ou prévenir une dégradation neurologique consécutive à une maladie neuro-dégénérative chez un être humain.

Dit autrement, l'invention porte sur une composition gazeuse, c'est-à-dire un médicament gazeux inhalable, à base de xénon pour une utilisation en combinaison avec au moins un antioxydant sous forme liquide ou solide pour traiter, ralentir ou prévenir une dégradation neurologique consécutive à une maladie neuro-dégénérative chez un être humain, l'antioxydant étant de la vitamine E ou du TROLOX.

Selon le cas, la combinaison ou composition médicamenteuse l'invention peut comprendre l'une ou plusieurs des caractéristiques techniques suivantes :
- elle contient entre 10 et 80% en volume de xénon.
- l'antioxydant est le TROLOX, c'est-à-dire l'acide 6-hydroxy-2,5,7,8-tétramethylchromane-2-carboxylique, qui est un analogue chimique hydrosoluble de la vitamine E (Davies et al, Biochem J., 1988).
- l'antioxydant est sous forme liquide.
- l'antioxydant est le TROLOX sous forme liquide.
- la maladie neuro-dégénérative est la maladie de Parkinson ou une maladie apparentée à la maladie de Parkinson, de préférence la maladie de Parkinson.
- le xénon gazeux est administré au patient avant, concomitamment ou après administration de l'antioxydant, de préférence après administration de l'antioxydant.
- la dégradation neurologique est liée à une entrée excessive d'ions Ca²⁺ dans l'une ou plusieurs populations de neurones vulnérables du patient ou être humain à traiter.
- le xénon est en mélange avec de l'oxygène et/ou de l'azote.
- la proportion d'oxygène est d'au moins 21% en volume.
- elle est constituée de xénon et d'oxygène ou de xénon, d'azote et d'oxygène.
- l'être humain est âgé de plus de 30 ans, de préférence d'au moins 40 ans, en particulier âgé d'au moins 50 ans.
- la proportion de xénon est d'au moins 20% en volume.
- la proportion de xénon est d'au plus 75% en volume, de préférence la proportion de xénon est d'au plus 60% en volume.
- le xénon administré au patient par inhalation.
- l'être humain traité ou à traiter, c'est-à-dire le patient, est un homme ou une femme.
- le xénon est utilisé, c'est-à-dire administré, en quantité ou proportion efficace.
- le médicament contient une quantité non anesthésique de xénon, i.e. sub-anesthésique.
- le xénon est mélangé avec de l'oxygène avant ou au moment de son inhalation par le patient, ou se présente sous forme d'un mélange gazeux « prêt à l'emploi » en pré-mélange avec de l'oxygène, et contient éventuellement un autre composé gazeux, par exemple de l'azote.
- le médicament est constitué d'un mélange gazeux formé d'oxygène et d'azote.
- le gaz est administré au patient, une ou plusieurs fois par jour.
- le gaz est administré au patient pendant une durée d'inhalation de quelques minutes à quelques heures, typiquement entre 15 minutes et 6 heures, préférentiellement moins de 4 heures.
- la durée, la posologie et la fréquence d'administration du xénon et/ou de l'antioxydant sont fonction de l'évolution de l'état neurologique du patient considéré et ces paramètres seront préférentiellement fixées par le médecin ou personnel soignant en fonction de l'état neurologique du patient considéré.
- le xénon (ou le mélange gazeux contenant le xénon) est conditionné dans une bouteille de gaz ayant une contenance (équivalent en eau) allant jusqu'à 50 litres, typiquement de l'ordre de 0,5 à 15 litres et/ou à une pression inférieure ou égale à 350 bar absolus, typiquement une pression comprise entre 2 et 300 bar. De préférence, la bouteille de gaz en acier, en aluminium ou en matériau composite, équipée d'un robinet ou d'un robinet à détendeur intégré permettant de contrôler le débit et éventuellement la pression du gaz délivré.
- lors du traitement, l'administration de xénon (ou du mélange gazeux contenant le xénon) au patient se fait par inhalation au moyen d'un masque facial ou nasal, de lunettes nasales ou au travers de tout autre système d'administration d'un gaz inhalable.

Plus précisément, dans le cadre de la présente invention, il a été mis en évidence que l'association de xénon et d'un antioxydant choisi parmi le TROLOX et la vitamine E, conduit à une action synergique de ces composés et qu'une telle association peut constituer un traitement prometteur des dégradations neurologiques résultant de maladies neurodégénératives, de type maladie de Parkinson.

En particulier, de bons résultats ont été obtenus en combinant le xénon avec du TROLOX ou acide 6-hydroxy-2,5,7,8-tétramethylchromane-2-carboxylique, qui est un analogue soluble de la vitamine E, lequel agit en inhibant le stress oxydatif et plus particulièrement en inhibant la peroxydation lipidique.

En effet, en opérant une telle combinaison, les inventeurs de la présente invention ont mis en évidence un effet synergique d'une association entre le xénon et l'antioxydant de type TROLOX, comme détaillé et exemplifié ci-après.

Une telle association Xe/antioxydant repose notamment sur les modes d'actions de ces types de composés ou molécules.

Ainsi, le xénon possède des propriétés inhibitrices des voies de signalisation glutamatergiques excitatrices (Dinse et al, Br J Anaesth, 2005), via son action antagoniste sur les récepteurs NMDA, mais aussi sur les récepteurs α-amino-3-hydroxy-5-méthylisoazol-4-propionate (AMPA), ainsi que sur les récepteurs kainate, qui composent les récepteurs ionotropiques au glutamate.

Par ailleurs, comme dit, le TROLOX agit en inhibant le stress oxydatif lié à la peroxydation des lipides (Guerreiro et al, J Neurochem, 2009).

Dès lors, en opérant une association combinée de xénon et d'antioxydant de type TROLOX ou vitamine E, les inventeurs ont mis en évidence une synergie d'action résultant d'un blocage des récepteurs glutamatergiques par le xénon et d'un effet antioxydant intracellulaire du TROLOX et de la vitamine E. En d'autres termes, les inventeurs de la présente invention ont constaté que le xénon permet de révéler ou de renforcer les effets bénéfiques de l'antioxydant par un effet synergique.

Décrite ici est aussi une méthode de traitement thérapeutique pour traiter, ralentir ou prévenir au moins une dégradation neurologique consécutive à une maladie neurodégénérative chez un patient humain, i.e. un être humain, dans laquelle :
i) on identifie un patient humain atteint d'une maladie neurodégénérative ou susceptible d'être atteint d'une telle maladie neurodégénérative,
ii) on administre par inhalation, audit patient, un médicament gazeux contenant du xénon, et
iii) on administre, audit patient, au moins un antioxydant sous forme liquide ou solide, de type TROLOX ou vitamine E, de manière à obtenir une protection neuronale et ainsi traiter, ralentir ou prévenir au moins une dégradation neurologique résultant de la maladie neurodégénérative chez ledit patient.

De préférence, à l'étape i) :
- le patient humain est un homme ou une femme.
- le patient humain est âgé de plus de 30 ans, de préférence d'au moins 40 ans, en particulier d'au moins 50 ans.
- l'identification du patient se fait par un médecin ou analogue.
- l'identification du patient se fait par examen technique de dépistage.
- la maladie neurodégénérative susceptible d'engendrer au moins une dégradation neurologique de type maladie de Parkinson, de préférence la maladie de Parkinson.
- ladite dégradation neurologique comprend une entrée excessive d'ions Ca²⁺ dans une ou plusieurs populations de neurones vulnérables du patient.

De préférence, à l'étape ii) :
- on administre l'antioxydant sous forme liquide.
- on administre au moins un antioxydant par voie entérale, i.e. la voie orale.
- on administre au patient du TROLOX (analogue de la vitamine E) ou de la vitamine E en tant qu'antioxydant.
- on administre au patient du TROLOX sous forme liquide.
- on administre, audit patient, au moins un antioxydant avant, concomitamment ou après inhalation du xénon par le patient.

De préférence, à l'étape iii) :
- la durée, la posologie et la fréquence d'administration du xénon sont choisies et/ou fixées en fonction de l'évolution de l'état neurologique du patient considéré.
- on administre une quantité efficace de xénon.
- on administre une quantité non anesthésique de xénon.
- on administre de 10 à 80% en volume de xénon, de préférence entre 20 et 50% en volume de xénon.
- on mélange le xénon avec de l'oxygène avant ou au moment de son inhalation par le patient, de préférence avec au moins 21 % en volume d'oxygène.
- on administre un mélange gazeux prêt à l'emploi constitué de xénon et d'oxygène (mélange binaire) ou de xénon, d'oxygène et d'azote (mélange ternaire).
- on administre le xénon gazeux une ou plusieurs fois par jour au patient.
- on administre le xénon gazeux au patient pendant une durée d'inhalation de quelques minutes à quelques heures, typiquement entre 15 minutes et 6 heures, préférentiellement moins de 4 heures.
- on administre le xénon gazeux au moyen d'un masque facial ou nasal, de lunettes nasales ou au travers de tout autre système ou dispositif d'administration de gaz à un patient.

L'invention va maintenant être mieux comprise grâce aux exemples suivants, donnés à titre illustratif et à la figure annexée qui montre les effets protecteurs synergiques du xénon et du TROLOX dans un modèle cellulaire mimant une dégénérescence parkinsonienne chronique.

### Exemples

Afin de démontrer l'efficacité de la combinaison du xénon et d'un antioxydant selon la présente invention, un modèle cellulaire de neurones dopaminergiques a été mis en place, dans lequel la mort neuronale est déclenchée en bloquant les systèmes de recapture au glutamate (GLU) grâce à un traitement prolongé avec du L-trans-pyrrolidine-2,4-dicarboxylic acid (PDC), un composé de synthèse dont le mécanisme d'action a été décrit précédemment par Zuiderwijk et coll, (Europ J Pharmacol, 1994).

La technique mise en oeuvre est décrite ci-dessous et les résultats obtenus sont illustrés sur la Figure annexée.

### Protocole d'obtention des cultures primaires de mésencéphale

Des cultures sont préparées à partir de mésencéphale d'embryons de rats, prélevés sur des rates gestantes Wistar, au jour 15,5 de gestation.

Le procédé d'obtention des cultures de mésencéphale comprend l'obtention d'une suspension cellulaire homogène par dissociation mécanique, c'est-à-dire non enzymatique, du tissu embryonnaire, en utilisant du milieu L15 de Leibovitz (Sigma Aldrich).

Des aliquotes de cette suspension sont ajoutés à des plaques Nunc de 48 puits, qui ont été préalablement revêtues d'une fine couche de polyéthylènimine (1 mg/ml, tampon borate pH 8,3) pour permettre l'adhésion des cellules neuronales (cf. Toulorge et coll, Faseb J, 2011).

La densité d'ensemencement est comprise entre environ 80000 et 100000 cellules/cm².

Les cultures de mésencéphale sont maintenues dans du milieu de culture Neurobasal, contenant un cocktail B27 sans antioxydant, du supplément N2, de la glutamine (2 mM) et un cocktail pénicilline/streptomycine (cf. Nafia et coll, J Neurochem, 2008). Le milieu et ses suppléments sont disponibles auprès de Life Technologies.

Jusqu'au moment où l'on évalue les effets des gaz d'intérêt, les cultures sont placées dans une enceinte conventionnelle thermostatée à 37°C, dans laquelle le CO₂ est maintenu à 5% en volume et où l'atmosphère est saturée en eau.

Aucun changement de milieu de culture n'est effectué pendant toute la période de la culture.

### Traitements pharmacologiques des cultures

Le processus dégénératif est déclenché par application d'un bloqueur des systèmes de recapture du glutamate, l'acide L-trans-pyrrolidine-2,4-dicarboxylique ou PDC (Zuiderwijk et al, Europ J Pharmacol, 1994).

L'antioxydant, à savoir du TROLOX, est ajouté aux cultures avant l'application de PDC.

Ces deux produits, i.e. PDC et TROLOX, sont disponibles commercialement auprès des sociétés RD Systems et Sigma Aldrich, respectivement.

### Maintenance des cultures sous atmosphère gazeuse contrôlée

Une fois les traitements pharmacologiques effectués, les boîtes multi-puits contenant les cellules en culture et la boite servant à l'humidification du compartiment interne de la chambre, sont placées sur une embase métallique qui reçoit la chambre d'incubation en Plexiglas. Les deux pièces (embase et chambre en Plexiglas) sont réunies par vissage, de manière jointive.

On injecte ensuite un mélange gazeux d'intérêt comprenant (% en volume) : 20 % d'O₂, 5 % de CO₂ et 75 % du gaz testé, dans la chambre d'incubation, avec valves d'entrée et de sortie ouvertes, tout en contrôlant le débit de sortie grâce à un débitmètre.

Les gaz testés sont de l'azote et du xénon.

Le débit de sortie de référence, fixé pour l'air à 10 litres/mn, est corrigé en fonction de la densité du mélange utilisé.

Lorsque la mesure du CO₂, atteint 5 % en sortie, on stoppe l'injection du mélange gazeux et la chambre est rendue totalement étanche en fermant les valves d'entrée et de sortie.

La chambre d'exposition est alors placée dans une enceinte à 37°C pendant toute la durée du protocole expérimental.

### Immunodétection de la tyrosine hydroxylase (TH) et comptages cellulaires

Après rupture de l'étanchéité par ouverture des valves d'entrée et de sortie et dévissage de la chambre de son embase, les cultures sont fixées avec 4% de formaldéhyde dans du PBS pendant 12 mn et ensuite incubées à 4°C avec un anticorps monoclonal anti-TH (dilution 1/5000) pendant 2 jours.

La révélation de cet anticorps est obtenue avec un second anticorps anti-souris IR DYE800 (EUROMEDEX, 1/10000 dans du PBS). L'acquisition des images est réalisée avec un imageur infrarouge de type ODYSSEY (Li-COR Biosciences) et la quantification des neurones se fait grâce au logiciel ICY (Institut Pasteur).

Les résultats obtenus dans le modèle de dégénérescence mésencéphalique par traitement chronique au PDC, révèlent des effets protecteurs additifs entre le xénon et le TROLOX qui sont résumés dans le Tableau suivant et représentés dans la Figure.

**Tableau : Résumé des principaux résultats de l'étude in vitro sur les cultures de mésencéphale**

| Cultures de mésencéphale analysées au jour (J) 16 | | |
|---|---|---|
| Traitements J 13 - J 16 (4 jours) | Mélange gazeux (20% O₂ + 5% CO₂ + 75 % de gaz testé) ; % en Vol. | Survie des neurones TH positifs |
| Groupe témoin I | N₂ | +++ |
| Groupe témoin II | Xe | +++ |
| Groupe PDC (100µM) | N₂ | - |
| Groupe PDC (100 µM) | Xe | ++ |
| Groupe PDC (100 µM) + TROLOX (0,1 µM) | N₂ | - |
| Groupe PDC (100 µM) + TROLOX (0,1 µM) | Xe | +++ |
| Groupe PDC (100 µM) + TROLOX (0,3 µM) | N₂ | - |
| Groupe PDC (100 µM) + TROLOX (0,3 µM) | Xe | +++ |
| Groupe PDC (100 µM) + TROLOX (1 µM) | N₂ | + |
| Groupe PDC (100 µM) + TROLOX (1 µM) | Xe | +++ |
| Groupe PDC (100 µM) + TROLOX (10 µM) | N₂ | ++ |
| Groupe PDC (100 µM) + TROLOX (10 µM) | Xe | +++ |

Dans le Tableau précédent :
- une réponse favorable, synonyme d'une baisse du niveau de mort des cellules neuronales en présence des traitements d'intérêt, est désignée par un signe « + », « ++ », ou « +++ » (+++ = niveau de référence).
- à l'inverse, une réponse défavorable est représentée par un signe « - », synonyme d'une augmentation de la mort neuronale.

Le TROLOX agit comme inhibiteur de la peroxydation lipidique et le xénon en bloquant les récepteurs NMDA.

Le PDC, induit quant à lui, un processus dégénératif en prévenant la recapture du glutamate produit et libéré de manière endogène par les cellules neuronales en culture, ce qui conduit à une sur-activation des récepteurs NMDA neuronaux et à une stimulation de la production intra-cellulaire d'espèces radicalaires de l'oxygène.

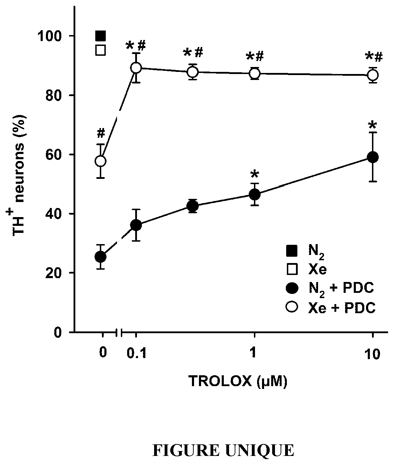

Au vu des résultats illustrés en Figure 1 annexée, on constate que la combinaison du xénon et d'un antioxydant, à savoir ici le TROLOX, conduit à un effet neuroprotecteur synergique comparé à l'effet de chaque molécule, prise séparément.

En fait, une réelle synergie d'action de l'association xénon/TROLOX se met en place et ce, de façon tout à fait inattendue.

Tout d'abord, ces essais ont montré que le PDC cause une perte importante des cellules neuronales dopaminergiques, à une concentration de 100 µM, sous une atmosphère contenant 75 vol.% d'azote. Cependant, ces effets délétères du PDC sont partiellement prévenus lorsque l'on remplace l'azote par le xénon puisque que le taux de survie passe alors de 25,4% à 57,7%.

De manière notable, le TROLOX qui n'a pas d'effet significatif dans ce modèle cellulaire lorsqu'il est appliqué seul à 0,1-0,3 µM, sous atmosphère contenant 75 % d'azote, devient efficace en présence de xénon, à savoir ici 75 % de xénon, permettant d'atteindre un taux de survie de l'ordre de 90 % dans ces conditions.

Une potentialisation des effets du xénon est aussi observée lorsqu'on applique des concentrations de TROLOX ≥ 1µM qui exercent un effet protecteur lorsqu'elles sont appliquées dans 75% d'azote.

Les résultats consignés sur la Figure ci-annexée, illustrent les effets protecteurs synergiques du xénon et du TROLOX dans un modèle cellulaire mimant une dégénérescence parkinsonienne chronique. Ces résultats ont été obtenus sur des cultures de mésencéphale de rat qui ont été traitées à la fin du jour 12 *in vitro* et pendant les 4 jours suivants, avec un inhibiteur de recapture du glutamate, le PDC (100 µM) sous atmosphère contenant 75 % d'azote (N₂ 75) ou 75 % de xénon (Xe 75), en présence ou non de TROLOX, testé de 0.1 à 10 µM.

Les cultures sont ensuite récupérées pour fixation et analyse. La survie neuronale est quantifiée dans les différentes conditions expérimentales testées, en comptant le nombre de corps cellulaires immuno-positifs pour le marqueur dopaminergique TH.

Les résultats sont exprimés en % (± SEM) des valeurs moyennes des cultures non-traitées au PDC, maintenues sous 75 % d'azote (condition témoin).

Ainsi, l'étude statistique faite par un test t de Student, en cas de comparaisons simples entre deux groupes ou par analyse de variance à un facteur ou méthode One-way ANOVA (ANalysis Of VAriance), suivie d'un test de Dunnett pour des comparaisons multiples effectuées par rapport à un groupe de référence (n= 9 pour chaque point expérimental) démontre que :
- dans une atmosphère à 75 % de xénon (vs 75 % d'azote dans la condition témoin), on note une augmentation de la survie des neurones dopaminergiques traités au PDC que du TROLOX soit présent ou non dans le milieu de culture (# p<0.05, vs cultures correspondantes sous 75 % d'azote, exposées au PDC, en présence ou non de TROLOX).
- dans une atmosphère à 75 % d'azote, le TROLOX produit un effet protecteur significatif à partir de 1µM et dans une atmosphère à 75 % de xénon, à partir de 0.1µM (* p<0.05, vs cultures exposées uniquement à 100 µM de PDC dans une même atmosphère gazeuse).

Le xénon conduit donc, lorsqu'il est associé à un antioxydant, de type TROLOX ou vitamine E, à un effet synergique dans le traitement, le ralentissement ou la prévention des dégradations neurologiques consécutives à une maladie neurodégénérative de type Parkinson, en particulier la maladie de Parkinson.

## Revendications

1. Combinaison médicamenteuse comprenant du xénon gazeux et au moins un antioxydant sous forme liquide ou solide pour une utilisation pour traiter, ralentir ou prévenir une dégradation neurologique consécutive à une maladie neurodégénérative chez un être humain, l'antioxydant étant de la vitamine E ou le TROLOX.

2. Combinaison médicamenteuse pour l'utilisation selon la revendication précédente, **caractérisée en ce qu'**elle contient entre 10 et 80% en volume de xénon.

3. Combinaison médicamenteuse pour l'utilisation selon l'une des revendications précédentes, **caractérisée en ce que** l'antioxydant est le TROLOX sous forme liquide.

4. Combinaison médicamenteuse pour l'utilisation selon l'une des revendications précédentes, **caractérisée en ce que** la maladie neurodégénérative est la maladie de Parkinson ou une maladie apparentée à la maladie de Parkinson.

5. Combinaison médicamenteuse pour l'utilisation selon l'une des revendications précédentes, **caractérisée en ce que** le xénon est mélangé à de l'oxygène.

6. Combinaison médicamenteuse pour l'utilisation selon l'une des revendications précédentes, **caractérisée en ce que** le xénon est mélangé à au moins 21% en volume d'oxygène.

7. Combinaison médicamenteuse pour l'utilisation selon l'une des revendications précédentes, **caractérisée en ce que** le xénon est mélangé à de l'oxygène et de l'azote.

8. Combinaison médicamenteuse pour l'utilisation selon l'une des revendications précédentes, **caractérisée en ce que** le xénon est mélangé à au moins 21% en volume d'oxygène.

9. Combinaison médicamenteuse pour l'utilisation selon l'une des revendications précédentes, **caractérisée en ce que** l'être humain est âgé de plus de 30 ans, de préférence d'au moins 40 ans, en particulier âgé d'au moins 50 ans.

10. Combinaison médicamenteuse pour l'utilisation selon l'une des revendications précédentes, **caractérisée en ce que** la proportion de xénon est d'au moins 20% en volume de xénon et d'au plus 75% en volume de xénon, de préférence de moins de 60% en volume de xénon.

## Patentansprüche

1. Arzneimittelkombination, umfassend gasförmiges Xenon und mindestens ein Antioxidans in flüssiger oder fester Form zur Verwendung bei der Behandlung, Verlangsamung oder Prävention der neurologischen Verschlechterung als Folge einer neurodegenerativen Erkrankung bei einem Menschen, wobei das Antioxidans Vitamin E oder TROLOX ist.

2. Arzneimittelkombination zur Verwendung nach dem vorstehenden Anspruch, **dadurch gekennzeichnet, dass** sie zwischen 10 und 80 Vol.-% Xenon enthält.

3. Arzneimittelkombination zur Verwendung nach einem der vorstehenden Ansprüche, **dadurch gekennzeichnet, dass** das Antioxidans TROLOX in flüssiger Form ist.

4. Arzneimittelkombination zur Verwendung nach einem der vorstehenden Ansprüche, **dadurch gekennzeichnet, dass** die neurodegenerative Erkrankung die Parkinson-Krankheit oder eine Erkrankung im Zusammenhang mit der Parkinson-Krankheit ist.

5. Arzneimittelkombination zur Verwendung nach einem der vorstehenden Ansprüche, **dadurch gekennzeichnet, dass** Xenon mit Sauerstoff vermischt ist.

6. Arzneimittelkombination zur Verwendung nach einem der vorstehenden Ansprüche, **dadurch gekennzeichnet, dass** Xenon mit mindestens 21 Vol.-% Sauerstoff vermischt ist.

7. Arzneimittelkombination zur Verwendung nach einem der vorstehenden Ansprüche, **dadurch gekennzeichnet, dass** Xenon mit Sauerstoff und Stickstoff vermischt ist.

8. Arzneimittelkombination zur Verwendung nach einem der vorstehenden Ansprüche, **dadurch gekennzeichnet, dass** Xenon mit mindestens 21 Vol.-% Sauerstoff vermischt ist.

9. Arzneimittelkombination zur Verwendung nach einem der vorstehenden Ansprüche, **dadurch gekennzeichnet, dass** der Patient über 30 Jahre alt ist, vorzugsweise mindestens 40 Jahre alt, insbesondere mindestens 50 Jahre alt.

10. Arzneimittelkombination zur Verwendung nach einem der vorstehenden Ansprüche, **dadurch gekennzeichnet, dass** der Anteil von Xenon mindestens 20 Vol.-% Xenon und höchstens 75 Vol.-% Xenon, vorzugsweise weniger als 60 Vol.-% Xenon beträgt.

## Claims

1. Drug combination comprising xenon gas and at least one antioxidant, in liquid or solid form, for use in treating, slowing, or preventing neurological damage following a neurodegenerative disease in a human being, the antioxidant being vitamin E or TROLOX.

2. Drug combination for the use according to the preceding claim, **characterised in that** it contains between 10 and 80% by volume of xenon.

3. Drug combination for the use according to one of the preceding claims, **characterised in that** the antioxidant is TROLOX in liquid form.

4. Drug combination for the use according to one of the preceding claims, **characterised in that** the neurodegenerative disease is Parkinson's disease or a disease related to Parkinson's disease.

5. Drug combination for the use according to one of the preceding claims, **characterised in that** the xenon is mixed with oxygen.

6. Drug combination for the use according to one of the preceding claims, **characterised in that** the xenon is mixed with at least 21% by volume of oxygen.

7. Drug combination for the use according to one of the preceding claims, **characterised in that** the xenon is mixed with oxygen and nitrogen.

8. Drug combination for the use according to one of the preceding claims, **characterised in that** the xenon is mixed with at least 21% by volume of oxygen.

9. Drug combination for the use according to one of the preceding claims, **characterised in that** the human being is over the age of 30 years old, more preferably at least 40 years old, in particular at least 50 years old.

10. Drug combination for the use according to one of the preceding claims, **characterised in that** the proportion of xenon is at least 20% by volume of xenon and at most 75% by volume of xenon, preferably less than 60% by volume of xenon.
